(19) **Europäisches Patentamt** **European Patent Office** **Office européen des brevets**

(11) **EP 4 018 924 A1**

(12) **EUROPEAN PATENT APPLICATION**

(43) Date of publication:
    **29.06.2022 Bulletin 2022/26**

(21) Application number: **21207579.0**

(22) Date of filing: **08.10.2014**

(51) International Patent Classification (IPC):
    **A61B 5/0537** (2021.01)       **A61B 8/08** (2006.01)
    **A61B 5/145** (2006.01)       **A61B 5/1477** (2006.01)

(52) Cooperative Patent Classification (CPC):
    **A61B 5/0537; A61B 5/14542; A61B 5/1477;**
    A61B 5/25; A61B 5/291

(84) Designated Contracting States:
    **AL AT BE BG CH CY CZ DE DK EE ES FI FR GB GR HR HU IE IS IT LI LT LU LV MC MK MT NL NO PL PT RO RS SE SI SK SM TR**

(62) Document number(s) of the earlier application(s) in accordance with Art. 76 EPC:
    **14188200.1 / 3 005 942**

(71) Applicant: **Aristotle University of Thessaloniki - Elke**
    **54636 Thessaloniki (GR)**

(72) Inventors:
    • **KARAPANTSIOS, Theodoros**
      **57013 Oreokastro Thessalonikis (GR)**
    • **EVGENIDIS, Sotiris**
      **57010 Chortiatis Thessalonikis (GR)**
    • **ZACHARIAS, Konstantinos**
      **55337 Triandria Thessaloniki (GR)**
    • **MESIMERIS, Theodoros**
      **54622 Thessaloniki (GR)**

(74) Representative: **Petsis, Christos**
    **4-6, Kyparissias**
    **542 49 Thessaloniki (GR)**

Remarks:
    This application was filed on 10.11.2021 as a divisional application to the application mentioned under INID code 62.

(54) **METHOD FOR THE DETECTION AND CHARACTERISATION OF BUBBLES IN LIQUIDS AND DEVICE THEREFOR, RESPECTIVELY SYSTEM**

(57)     The invention concerns an ultra-sensitive and accurate electrical impedance spectroscopy technique for the detection and characterization of bubbles in liquids that is required in numerous industrial processes. The method is based on measuring non-invasively the electrical impedance variation of two-phase mixtures: liquid as conductive phase, resp. gas as non-conductive phase, applying an AC excitation signal of the proper frequency value identified by preliminary frequency scanning. The system takes advantage of innovative signal acquisition (hardware) device, and digital signal processing (software) methods and manages to detect accurately for the first time ever the presence of a few bubbles corresponding to extremely low volumetric gas fraction values, up to < 0,1%. Also, the high measurement sensitivity of 0,001 % in conjunction with advanced signal processing provides valuable qualitative and quantitative information on bubble size characteristics and allows for correlating bubble size to the spectral content of acquired signals.

FIG. 1

EP 4 018 924 A1

**Description**

FIELD OF THE INVENTION

The invention relates to a method for the detection and characterization of particles, especially bubbles in liquids for carrying out said system with method.

BACKGROUND OF THE INVENTION

The presence of bubbles in liquids is encountered in a variety of industrial processes including chemical and petroleum processing, oil and gas extraction and transportation, and nuclear power generation, up to the human's blood circulation during Decompression Sickness notably among astronauts, scuba divers and metro workers, which may cause undesired effects. A reliable, non-invasive bubbles detection and characterization is then of particular importance both for the control and improvement of industrial processes, and also for the protection of human health.

Existing non-invasive techniques for detecting and characterizing bubbles in liquids can be divided into three main categories : optical, acoustic and electrical.

The optical measuring techniques are widely used to measure the bubble size, including photographic techniques and techniques based on scattering and diffraction of light. The main disadvantages of optical techniques are two: their use is limited to transparent liquids and measurement of low volumetric gas fractions. In addition, the imaging techniques including X-Ray, Gamma Ray, MRI, yet provide excellent spatial resolution but the presence of intense radiation and magnetic fields, the use of cryogenic liquids, such as liquid helium and liquid nitrogen, and slow time response make them impractical in industrial applications.

PRIOR ART

As regards the acoustic techniques, they are divided into passive and active. Passive techniques do not have wide application because it is very difficult to extract information from the acoustic wave generated from gas phase flow. In that sense, the pressure measurement techniques are considered passive as well. The LG01 by SENSIRION, US-6,813,944 B2 is a non-invasive passive pressure measurement sensor, but not a complete integrated system. However, it is employed in limited industrial applications due to a low flow rate measurement range. The detection range is limited to high volumetric gas fraction changes only, while the threshold of detection is unspecified. Also, said LG01 system has the drawback not to provide any information regarding the bubble size.

Active acoustic techniques, on the other hand, are based on measuring the propagation speed and attenuation of acoustic waves in liquids. The presence of bubbles affects both the speed and attenuation, and this effect is more pronounced with the increase of volumetric gas fraction. Their main drawback is the lack of reliable measurements for volumetric gas fraction values higher than 0,1%, resulting in a very high attenuation, making active acoustic techniques impractical for industrial applications. Even when reliable measurements are conducted, the lack of rigorous mathematical modelling as well as numerical solving limitations prohibit the interpretation of acoustic signals in terms of bubble size detection and characterization. Besides, the necessary hardware is quite complex for accurate measurements.

Referring to US 7,661,293 & 7,661,294, the AD-101 by Cosense Inc. is an active acoustic measurement sensor, but neither a complete integrated system however, which is based on transmitting and receiving ultrasonic waves. It is applied for detecting bubbles non-invasively, but its sensitivity is unspecified. Moreover, it does not provide information regarding the bubble size and it cannot be employed for pipe diameters larger than 25 mm. Also, the continuous wave (CW) or pulsed wave (PW) medical ultrasound systems as well as Doppler techniques are sorted in active acoustic techniques category. However, these ultrasound systems provide mainly qualitative information, whereas quantitative estimation of bubble size is problematic because of several technical drawbacks, which is thus unsatisfactory.

A third category is directed to the electrical techniques, which are based on measuring the impedance, the capacitance or equivalently the electric permittivity, or the conductance -or inversely the resistance- of the liquid. The conventional electrical techniques, when applied in industrial applications, provide reliable results essentially for high values of volumetric gas fraction, i.e. above 10%, which again does not provide a comprehensive technique, thus leaving the problem unsolved for lower fractions.

AIM OF THE INVENTION

The present invention aims at remedying the lacks set out above by providing a solution to the problem including for both the former higher fractions and the latter lower fractions, thus tending to a complete integrated system.

## SUMMARY OF THE INVENTION

For that purpose, there is provided according to the invention a method for the detection and characterisation of particles such as bubbles, droplets, solids in liquids, comprising the steps of:

    a) applying at least 2 electrodes in a manner to obtain electrical impedance measurements
    b) generating an electrical signal from about 1 kHz to about 96 kHz, and
    c) acquiring electrical signals through said electrodes by means of data acquisition means, particularly of the card type,
    d) calculating impedance time series of said electrical signals by applying digital signal processing, wherein said method is remarkable in that it further comprises the steps of
    e) generating said electrical signal at various frequencies by using frequency scanning through said electrodes and
    f) real time monitoring by means of a personal computer, storing and digital processing of said time series, particularly for industrial applications in in-vitro, wherein particles (solids, bubbles, droplets) are detected, and their size and volumetric concentration are estimated. Thanks to the invention, there is thus provided an innovative, highly sensitive and accurate, non-invasive electrical impedance spectroscopy technique, wherein said method proposed according to the invention allows detecting accurately for the first time even the presence of a few particles, esp. bubbles, corresponding to extremely low volumetric gas fraction values.

According to a particular embodiment of the invention, said method comprises the step consisting in that the velocity of said particles (bubbles, droplets, solids) is estimated and the presence of a few particles corresponding to extremely low volumetric concentration values is detected up to less than 0,1 %.

According to a preferred embodiment of the invention, a high measurement sensitivity of up to 0,001%, is performed in conjunction with a selected, resp. appropriate signal analysis, wherein a qualitative and quantitative information on particle (bubble, droplet, solid) size characteristics is provided, wherein said generation of electrical signals is performed with a voltage source or with a current source.

Particularly, the reactance X, as imaginary part of the impedance $Z_m$ is minimized by reducing $Z_m$ to a small amount compared to the resistance R, as real component of $Z_m$, whereas the phase shift of the output relative to the input current is neglected, wherein the reactance is made less than 1/10 of the resistance

$$\frac{X}{R} \leq \frac{1}{10}$$

whereas the magnitude of the impedance is:

$$Z = \sqrt{R^2 + X^2} = \sqrt{R^2 + \frac{R^2}{100}} = R\sqrt{1,01} = 1,0049R \approx R$$

yielding that the impedance is transformed to resistance and Equation (2) is reduced to:

$$R_m = (k^{-1} - 1)R_t$$

yielding by equivalence, the corresponding phase shift requirement as:

$$\varphi \leq tan^{-1}\left(\frac{X}{R}\right) = tan^{-1}\left(\frac{1}{10}\right)$$
$$-6° \leq \varphi \leq 6°,$$

wherein the phase response analysis is independent from the excitation technique and the kind of source voltage or current, more particularly whereby a frequency scanning is conducted to identify the phase response of the liquid with respect to frequency, by applying digital signal analysis and the phase shift is calculated for any discrete frequency, revealing the phase response of the liquid, which is followed by setting the excitation frequency of the signal generator in the frequency range that satisfies the phase shift requirement of the latter Equation, therefore indicating the range of maximum bubble detection sensitivity, which is thus provided thanks to the invention.

According to a further embodiment of the invention, at least two electrodes of wire, ring, or plate type are applied.

Also, the high measurement sensitivity of 0,001% in conjunction with the appropriate signal analysis provides valuable qualitative and quantitative information on bubble size characteristics. Furthermore, the use of advanced signal processing techniques allows for correlating bubble size with the spectral content of acquired signals.

According to a still further embodiment of the invention, the spectral content of said acquired signals is thus determined and an advanced signal processing technique is carried out, wherein the particle (bubble, droplet, solid) size is correlated with the spectral content of said acquired signals, wherein for the analysis of measured signals, impedance time series are calculated by applying digital signal processing and statistical analysis, including any one of auto-correlation, cross-correlation, spectrogram analysis, continuous and discrete transform and Hilbert-Huang transform techniques, resp. wherein a cross-correlation of the signals between two electrode pairs is carried out providing bubble velocity.

According to an even further embodiment of the invention, each electrode of said at least two electrodes in in-vitro applications is non-intrusively flush mounted to the inner the pipe, notably cylindrical or with rectangular walls.

According to a yet further embodiment of the invention, said method comprises the step of non-intrusive estimation of the concentration of each liquid phase in a flow consisting of two immiscible liquids.

According to a specific embodiment of the invention, said method is used for the study and development of emulsion stability and foam drainage that interests food industry and cosmetics.

According to another embodiment of the invention, said method consists of a crack or/and air pocket detection as well as relevant humidity estimation in construction materials, wherein two or more plate electrodes are placed on the surface of the material under test for conducting electrical measurements.

This invention also relates to a novel electrical impedance spectroscopy device for the detection and characterisation of particles including bubbles, droplets and/or solids, in liquids, that is remarkable in that it comprises in combination:

a) an electrical signal generator means,
b) at least two electrodes means,
c) a data acquisition device means,
d) a personal computer means for real time monitoring, storing and digital processing of acquired signals.

Particularly, said device further comprises a voltage source and a termination resistor for providing adjustment of the measurement sensitivity by means whereof said electrical signal is generated or
a current source and a current sense resistor by means whereof said electrical signal is generated and/or

it comprises a voltage controlled current source by means whereof said electrical signal is generated by means of an electronic circuit.

According to a particular embodiment of said device according to the invention, the data acquisition device is provided with a resolution of 24 bit and a sampling frequency of 192 kS/s by means whereof said device measures gas fractions of 0,1% with a measurement sensitivity of 0,001 %.

According to a more particular embodiment of the device according to the invention, there is provided an electrical impedance spectroscopy device for carrying out a method wherein detection means are provided for a non-invasive particles (bubbles, droplets, solids) detection as well as for the estimation of their size and corresponding volumetric concentration, which is remarkable in that it comprises:

- at least two non-intrusive sensors, particularly flush-mounted ring, plate or another type of electrodes depending on the pipe cross-section such as circular or rectangular, for conducting electrical measurements;
- a data acquisition means consisting of a data acquisition card of high sampling rate and resolution for acquiring electrical impedance measurements;
- monitoring means consisting of a PC for real time monitoring, storing and digital processing of acquired signals.

According to an even more particular embodiment of the device according to the invention, there is provided a system for the detection and characterization of bubbles in liquids required in an industrial process wherein it involves an electrical impedance spectroscopy device as defined above, for carrying out a method as defined above, wherein said method is based on the electrical impedance variation of two-phase mixtures - liquid as conductive phase, resp. gas as non-conductive phase - which is measured by non-invasively applying an AC excitation signal of a proper frequency value identified by preliminary frequency scanning, wherein said system combines signal acquisition (hardware) device, and digital signal processing (software) methods, thereby managing to detect accurately the presence of a few bubbles corresponding to extremely low volumetric gas fraction values, up to < 0,1%, with a high measurement sensitivity of 0,001 % in conjunction with advanced signal processing, thereby providing valuable qualitative and quantitative information on bubble size characteristics and allowing for correlating bubble size to the spectral content of acquired signals.

According to a particular embodiment of the system according to the invention, there is provided a system including an electrical impedance spectroscopy device for carrying out said method that is categorized to electrical techniques measuring the electrical impedance, wherein the electrical impedance technique involved consists of a combination of hardware in accordance with said device, on the one hand, and a corresponding program code as a software for digital processing of measurements in accordance with said method, on the other hand. The electrical quantities that are measured and taken into account are the resistance -or inversely the conductance- and the reactance, or both of them, i.e. the impedance, wherein it is based on measuring the variation of said electrical impedance of the two-phase mixture, consisting of a liquid being a conductive phase, resp. gas as a non-conductive phase, by applying a high frequency alternating electric current (AC) or voltage via said electrodes.

Particularly, the next step consists of the way that the acquired signals are digitally processed employing custom made software, that complements the said hardware of said electrical impedance technique, wherein measurements of low amplitudes are conducted, wherein unwanted interference including power line related noise are rejected, wherein the recorded AC signals $V_{E1}$ and $V_{E2}$ or $V_{E1}$-$V_{E2}$ are digitally filtered, thereby applying a very selective digital band pass filter centred at the excitation frequency with a nominal narrow bandwidth, of preferably about 3 kHz, thereby rejecting said electrical interferences, resulting in high quality electrical signals regardless of liquid physical properties, phase velocities and bubble sizes. More particularly, the bandwidth is set at a desired value, depending on the application, by changing the program code, wherein a narrow filter bandwidth is used, which eliminates the said power line related noise and also practically suppresses all extraneous noise outside the band pass frequency range, thereby reducing the total noise power and allowing to accurately measure very low voltage differentials, down to a few $\mu$V.

Still more particularly, the next step in said signal processing consists of the accurate extraction of acquired signals envelope, whereby the envelope of the filtered AC signals is digitally extracted, virtually without any signal loss, wherein the process is non-linear and thus creating high frequency spurious components, which are removed by applying a low pass filter with a nominal cut-off frequency, particularly of about 100 Hz.

According to a more particular embodiment of the system according to the invention, the low pass filtered envelope of the signals $V_{E1}$ and $V_{E2}$, or the differential voltage $V_{E1}$ - $V_{E2}$, constitutes the real peak voltage amplitude of the signals without any loss of information or distortion,_after which the impedance's magnitude of two-phase medium ($Z_m$) is calculated employing Equations (2) and (13),

$$Z_m = (k^{-1} - 1)R_t \qquad (2)$$

wherein $Z_m$ is the equivalent impedance as measured by the two electrodes, resp.

$$Z_m = \frac{V_{E1} - V_{E2}}{I_{out}} = \frac{V_m}{I_{out}} \qquad (13)$$

wherein the measured impedance -or inversely measured admittance- as sensed by the electrodes (E1) and (E2), is the magnitude of $Z_m$ and is calculated as the ratio of the differential voltage -voltage drop- between (E1-E2) and output current; wherein the $Z_m$ values are compared prior and after gas phase entrance in the liquid phase, by means whereof detected bubbles are quantitatively inside the two-phase medium, while the resulted time series is converted to volumetric gas fraction time series, in particular for the case of a pure resistive medium, such as a two-phase flow inside a pipe for a predefined excitation frequency, wherein standard conversion models notably of Maxwell and/or Begovich are employed, wherein impedance time series carry crucial information in both frequency and time domain, wherein several statistical and spectral features are associated with bubble size characteristics, wherein various signal processing and statistical analysis techniques are applied to the resulting time series, such as

a) 1st, 2nd and higher order moments including Mean value, standard deviation, coefficient of variation, skewness and kurtosis; or
b) a Fast Fourier Transform (FFT) spectral analysis algorithm,

wherein said signal analysis techniques are applied on the impedance time series in order to study bubble size features for specific two-phase flow applications;

particularly wherein, depending on the application notably in-vitro, different quantities are determined by the said statistical and spectral tools such as skewness, standard deviation, power spectral density, dominant spectral frequency, or a combination of them, which quantities provide information on bubble size distribution, as well as to be correlated with bubble size;
preferably wherein a cross-correlation of the signals between two electrode pairs is carried out providing bubble velocity.

According to a more particular embodiment of the system according to the invention, said electrical impedance technique software of digital processing of the acquired signals is carried out either in real time or at a later time as post-processing, wherein new processing features are added by changing the program code, wherein storage capacity, memory and CPU processing power are provided for the storage of numerous voluminous data files being accomplished, and the volume of the acquired files is determined by the recording duration which is dictated by the stochastic features of the subject two-phase flow.

Further features and properties of the device and the method according to the invention are defined in the further appended subclaims, notably including a device for carrying out said method that is based on measuring non-invasively with exceptional accuracy and sensitivity the electrical impedance variation of two-phase mixtures, wherein liquid is a conductive phase, and gas a non-conductive phase. For this, preliminary frequency scanning identifies the proper frequency value of the applied AC excitation signal in terms of measurement sensitivity and accuracy. Additionally, innovative signal acquisition, as hardware, and digital signal processing, as software methods are applied to improve measurement sensitivity several orders of magnitude compared with conventional electrical techniques. Said device according to the invention is remarkable in that detection means are provided therein for a non-invasive bubbles detection as well as estimation means for the estimation of their size and corresponding gas volumetric fraction, notably in decompression chambers. Said device comprises at least two non-invasive sensors, particularly electrodes for being attached; a data acquisition card of high sampling rate and resolution for acquiring electrical impedance measurements; a PC for real time monitoring, storing and digital processing of acquired signals.

Besides, at least two flush-mounted ring electrodes are provided for conducting electrical measurements for non-intrusive bubbles detection.

Particularly, at least two flush-mounted ring, plate or any other type of electrodes depending on the pipe cross-section circular, rectangular, are provided for conducting electrical measurements for non-intrusive bubbles detection, bubble size and gas volumetric fraction estimation as well as flow regime transition identification in gas-liquid two-phase flows found in several industrial applications. In addition, a set of pipes is provided therein for fluids' transport and wherein the presence of gas is of great significance, notably in food industry, petroleum lines, product line of polymers, comparing to the previous claims. At least two flush-mounted rings, plate or any other type of electrodes depending on the pipe cross-section circular, rectangular, etc. are provided for conducting electrical measurements.

Further, it includes means for non-intrusive solid particles detection, particles size and solid concentration estimation in solid-liquid two-phase flows found in several industrial applications, e.g. food industry.

Still further, it includes means for a non-intrusive estimation of the concentration of each liquid phase in a flow consisting of two immiscible liquids.

Yet further, it includes means for the study of emulsion stability and foam drainage that interests food industry and cosmetics.

Additional features of the method and the device according to the invention are set out hereafter, notably an electrical impedance spectroscopy method for non-invasive bubbles detection as well as estimation of their size and corresponding gas volumetric fraction in decompression chambers, comprising the steps of attaching at least two non-invasive sensors, particularly electrodes, notably including ElectroCardioGraphy (ECG) or ElectroEncephaloGraphy (EEG) pads; acquiring electrical impedance measurements by means of a data acquisition card of high sampling rate and resolution; real time monitoring by means of a PC, storing and digital processing of acquired signals, thanks to which there is thus provided an innovative, highly sensitive and accurate, non-invasive electrical impedance spectroscopy technique;
particularly, a method allowing detecting accurately for the first time even the presence of a few bubbles corresponding to extremely low volumetric gas fraction values, up to less than 0,1%.

Also, the high measurement sensitivity of 0,001% in conjunction with the appropriate signal analysis provides valuable qualitative and quantitative information on bubble size characteristics.

Furthermore, the use of advanced signal processing techniques allows for correlating bubble size with the spectral content of acquired signals.

Preferably, said method comprises the steps of a non-intrusive bubbles detection, bubble size and gas volumetric fraction estimation as well as flow regime transition identification in gas-liquid two-phase flows found in several industrial applications, by means of a set of pipes that is incorporated for fluids' transport and wherein the presence of gas phase is of great significance, such as in the food industry, petroleum lines, product line of polymers, wherein at least two flush-mounted ring, plate or any other type of electrodes depending on the pipe cross-section circular, rectangular, are incorporated for conducting electrical measurements.

In particular, said method comprises the steps of non-intrusive solid particles detection, particles size and solid concentration estimation in solid-liquid two-phase flows found in several industrial applications, such as in food industry.

More particularly, said method comprises the step of non-intrusive estimation of the concentration of each liquid phase in a flow consisting of two immiscible liquids.

Yet more particularly, said method is for the study of emulsion stability and foam drainage that interests food industry and cosmetics.

Another method consists of a crack or/and air pocket detection as well as relevant humidity estimation in construction materials, wherein two or more plate electrodes are placed on the surface of the material under test for conducting electrical measurements.

Yet another method consists of an accurate, multi-frequency measurement of liquids' electrical conductivity. In this case, two plate electrodes are immersed in the test liquid and placed opposite each other for conducting electrical measurements.

Also a novel electrical impedance spectroscopy device is proposed for carrying out said method that is based on measuring non-invasively with exceptional accuracy and sensitivity the electrical impedance variation of two-phase mixtures, wherein liquid is a conductive phase, and gas a non-conductive phase. For this, preliminary frequency scanning identifies the proper frequency value of the applied AC excitation signal in terms of measurement sensitivity and accuracy. Additionally, innovative signal acquisition, as hardware, and digital signal processing, as software methods are applied to improve measurement sensitivity several orders of magnitude compared with conventional electrical techniques. Said device according to the invention is remarkable in that detection means are provided therein for a non-invasive bubbles detection as well as estimation means for the estimation of their size and corresponding gas volumetric fraction, in decompression chambers. Said device comprises at least two non-invasive sensors, particularly electrodes for being attached, such as ElectroCardioGraphy (ECG) or Electro-EncephaloGraphy (EEG) pads; a data acquisition card of high sampling rate and resolution for acquiring electrical impedance measurements; a PC for real time monitoring, storing and digital processing of acquired signals.

In particular, said device comprises at least two flush-mounted ring, plate or any other type of electrodes depending on the pipe cross-section circular, rectangular, which are provided for conducting electrical measurements for non-intrusive bubbles detection, bubble size and gas volumetric fraction estimation as well as flow regime transition identification in gas-liquid two-phase flows found in several industrial applications. In addition, a set of pipes is provided therein for fluids' transport and wherein the presence of gas phase is of great significance, notably in food industry, petroleum lines, product line of polymers, wherein at least two flush-mounted rings, plate or any other type of electrodes depending on the pipe cross-section circular, rectangular, etc. are provided for conducting electrical measurements.

Remarkably, the device includes means for accurate, multi-frequency measurement of liquids' electrical conductivity. In this case, two plate electrodes are immersed in the test liquid and placed opposite each other for conducting electrical measurements.

Further details of the device and the method according to the invention will emerge from the following detailed description of some embodiments of the invention, which are illustrated with the aid of the attached drawings.

BRIEF DESCRIPTION OF THE DRAWINGS

FIG. 1 presents the design logic of the electrical impedance technique applying the voltage source excitation mode for the detection and characterization of bubbles in liquids.

FIG. 2 shows the electrical equivalent of the impedance technique applying the voltage source excitation mode.

FIG. 3 presents the design logic of the electrical impedance technique applying the current source excitation mode for the detection and characterization of bubbles in liquids.

FIG. 4 shows the electrical equivalent of the impedance technique applying the current source excitation mode.

FIG. 5 demonstrates the circuit schematic of the novel Howland modified Voltage Controlled Current Source (VCCS), for AC voltage sources as an input.

FIG. 6 depicts a perspective cut-away, cross section view of the flush mounted ring electrodes to the inner walls of a cylindrical dielectric (non-electrical conductive) pipe section.

DESCRIPTION

The proposed invention is categorized to the electrical techniques measuring the electrical impedance. The electrical impedance technique consists of the hardware and the corresponding program code as a software for digital processing of measurements. The electrical quantities that are measured and taken into account are the resistance -or inversely the conductance- and the reactance or both of them, i.e. impedance. It is based on measuring the variation of electrical impedance of the two-phase mixture, i.e. liquid being a conductive phase, resp. gas as a non-conductive phase, by applying a high frequency alternating electric current (AC) or voltage via properly designed electrodes.

Electrodes geometry varies with the application. When the technique is applied in-vitro for bubbles detection inside a pipe, then ring, plate or any other type of electrodes may be used depending on the pipe cross-section, e.g. circular, rectangular, etc.. In any case, the electrodes are flush mounted to the inner walls of the pipe in order to provide non-intrusive measurements avoiding any undesired flow disturbance. The proper selection of electrodes' dimensions and separation distance is a matter of concern and thus is taken seriously into account, as these two parameters affect strongly the measuring volume inside the pipe as well as the spatial resolution.

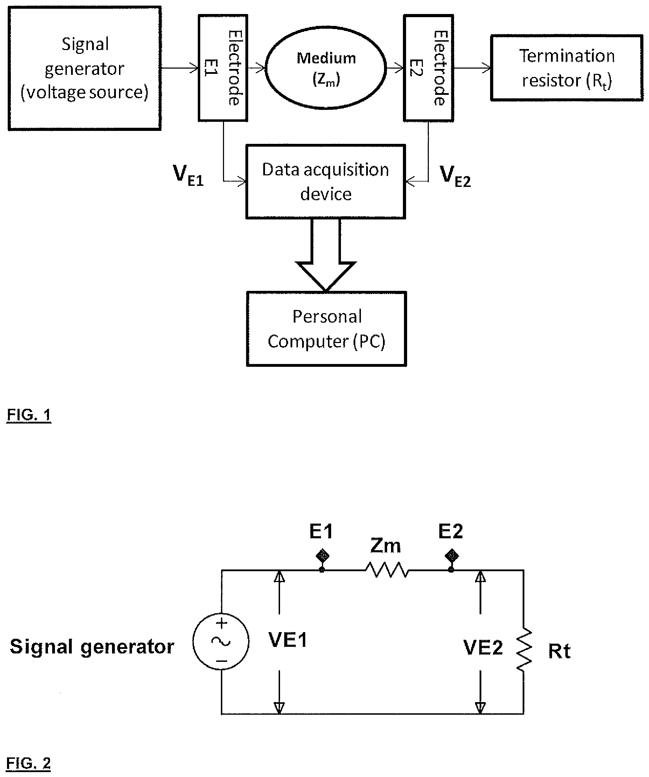

As mentioned above, there are two applicable excitation modes: voltage source, when the applied voltage is set at a defined value (voltage control), and current source, when the applied current is set at a defined value (current control). Fig. 1 demonstrates the design logic for measuring the electrical impedance of a gas/liquid mixture $Z_m$, or inversely the electrical admittance $Y_m$, applying the voltage source excitation mode. Moreover, Fig. 2 shows the electrical equivalent of the measurement system presented in Fig. 1. $V_{E1}$ is the input voltage that excites electrically the medium, connected to the input electrode E1 generating an input current passing through the medium. The hardware provides the optimum adjustment of AC voltage -or AC current when a current source is applied- in terms of amplitude and frequency employed to the respective electrodes which are placed either inside a pipe or above the human skin. Regardless the excitation mode -voltage or current- and application - notably in-vitro -, preliminary testing reveals the proper excitation frequency and amplitude that provide the highest measurement sensitivity and accuracy. Also, the hardware of the novel electrical impedance technique allows for employing different waveform types of excitation signal to the electrodes -continuous: sinusoidal, square, triangular, etc., or pulsed-, depending on the application requirements. The input current generated by the excitation voltage $V_{E1}$ creates a voltage drop due to the finite, but time variable, impedance $Z_m$ of the medium, and exits the section from the output electrode E2. The output electrode is connected to the one end of the termination resistance $R_t$, while the other end of the resistance is connected to signal generator (common voltage point). If needed, the common point could be grounded. The voltage across the termination resistance is the measured output voltage $V_{E2}$. $Z_m$ and $R_t$ constitute a voltage divider and the ratio k of the voltages is:

$$k = \frac{V_{E2}}{V_{E1}} = \frac{R_t}{Z_m + R_t}$$

(1)

Solving for $Z_m$, it is obtained:

(2)

$$Z_m = (k^{-1} - 1)R_t$$

$Z_m$ is the equivalent impedance as measured by the two electrodes. Equation 1 can be written in the following complex exponential form:

$$\frac{R_t}{|Z_m|e^{j\phi_m} + R_t} = |k|e^{j\phi_k}$$

(3)

where $|Z_m|$ and $\phi_m$ represent the magnitude and the phase of $Z_m$ respectively and suchlike $|k|$ and $\phi_k$ are the magnitude and the phase of k. It stands from the electric circuit's theory that any impedance can be resolved in Cartesian form into a real and an imaginary component $Z = R + jX$, i.e. resistance and reactance respectively. Therefore, any impedance can be mathematically represented by a complex number and the imaginary component is modelled depending on the sign by either an inductor or a capacitor, which are frequency selective components. Consequently, the voltage ratio k will be a complex number and a function of frequency. In a conductive medium, the bubbles affect the resistance of the medium and any variation of the resistance, due to bubbles presence, is compared with the reactive component of the impedance. Therefore, the effect of bubbles on the magnitude of the impedance $Z_m$ is reduced and as a result the bubble detection sensitivity deteriorates. In order to obtain the maximum possible bubble detection sensitivity, the reactance (imaginary part) of $Z_m$ has to be ideally zero. In practice, if the reactance X (imaginary component) is small compared to the resistance R (real component), then the phase shift of the output relative to the input current can be neglected. This requirement is fulfilled when the reactance is less than 1/10 of the resistance:

$$\frac{X}{R} \leq \frac{1}{10} \qquad (4)$$

Therefore, based on Equation (4) the magnitude of the impedance is:

$$Z = \sqrt{R^2 + X^2} = \sqrt{R^2 + \frac{R^2}{100}} = R\sqrt{1.01} = 1.0049R \approx R \qquad (5)$$

From Equation (5) it results that if the requirement of Equation (4) is fulfilled, then the impedance is transformed to resistance and Equation 2 is reduced to:

$$R_m = (k^{-1} - 1)R_t \qquad (6)$$

Equivalently, the corresponding phase shift requirement can be written as:

$$\phi \leq tan^{-1}\left(\frac{X}{R}\right) = tan^{-1}\left(\frac{1}{10}\right) \qquad (7)$$

$$-6° \leq \phi \leq 6° \qquad (8)$$

It is underlined that the phase response analysis is independent of the excitation technique and the kind of source -voltage or current- as well. There is a possibility of some imaginary component existence, even in a purely resistive medium, due to stray capacitances of the connection wires or electrodes, in this case, considering that an air bubble is dielectric, the maximum bubble detection sensitivity is accomplished when there is no phase shift in the excitation current. Therefore, if needed, a frequency scanning can be conducted to identify the phase response of the liquid with respect to frequency. This is accomplished by applying digital signal analysis and the phase shift can be calculated for any discrete frequency, revealing the phase response of the liquid. This allows setting the excitation frequency of the signal generator in the frequency range that satisfies the phase shift requirement of Equation (8), therefore indicating the range of maximum detection sensitivity.

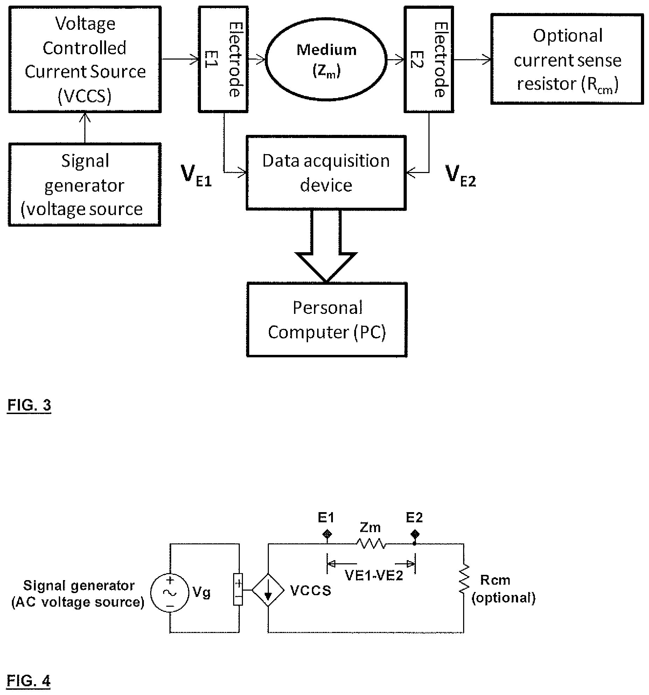

The current source excitation mode is accomplished by applying an alternating current Voltage Controlled Current Source (VCCS). The VCCS is located between the voltage source and the electrode E1, as shown in Fig. 3. Fig. 4 shows the electrical equivalent of the measurement set-up shown in Fig. 3. For the needs of the excitation, a proper AC current source (VCCS) of the desired frequency and amplitude is applied. $V_g$ is the output voltage of the signal generator, $V_m = V_{E1} - V_{E2}$ is the differential voltage measured across the electrodes E1 and E2 caused by the excitation current of the VCCS, $Z_m$ is the measured impedance between the two applied electrodes and $R_{cm}$ is an optional resistor for current sensing. The VCCS is a component that converts any type of voltage source into a current source. The current is controlled by the voltage applied at the input. This feature is the great advantage of VCCS compared with a current source generator. Instead of using a current generator where the available waveform type would only be sinusoidal, the VCCS can convert any arbitrary waveform voltage. For example, continuous waves such as sinusoidal, square, triangular, or pulse waves of any voltage amplitude and frequency can be converted. Although the vast majority of applications for the detection and characterization of bubbles in liquids would require a sinusoidal waveform, any other waveform type can be employed and tested. Since the output of the VCCS is current and the input is voltage, therefore the transfer function, which is the output over the input, is in terms of $\Omega^{-1}$. These components are also called as transconductance devices and the most commonly used are the transconductance amplifiers (TA). The transconductance amplifier is like a typical voltage amplifier but the amplified output is a current. It is an amplifier because the magnitude of the transfer function is $|H_{TA}| = \frac{I_o}{V_i} > 1$, where $I_o$ is the output current and $V_i$ is the input voltage. For the application of the impedance technique in liquids, the excitation current does not have to be greater than a few mA. Also, it shall be taken into account that many biomedical devices operate with a stimulation current of 1mA RMS or less, e.g. 0,1 mA, as the present electrical impedance technique intends to be applied for bubbles detection in humans' bloodstream as well. As a result, the output of the voltage source would be in the order of a few mV, which is not practically possible. The practical implementation of the VCCS requires the current output to be in mA and the voltage input in V, therefore the magnitude of the transfer function of VCCS $|H_{VCCS}|$ would be far less than one:

$$|H_{VCCS}| = \frac{I_o}{V_i} = \frac{mA}{V} \ll 1 \qquad (9)$$

Hence, the VCCS is not a TA but a transconductance attenuator. After worldwide survey, there is no commercial off-the-shelf device that fulfils the requirements of VCCS as a transconductance attenuator, in terms of attenuation, frequency response, distortion, noise, etc. Therefore, a custom made VCCS circuit is designed in order to fulfil the requirements for the detection and characterization of bubbles in liquids. A practical value of the $|H_{VCCS}|$ or gain would be:

$$|H_{VCCS}| = \frac{1mA}{1V} = 0,001 \qquad (10)$$

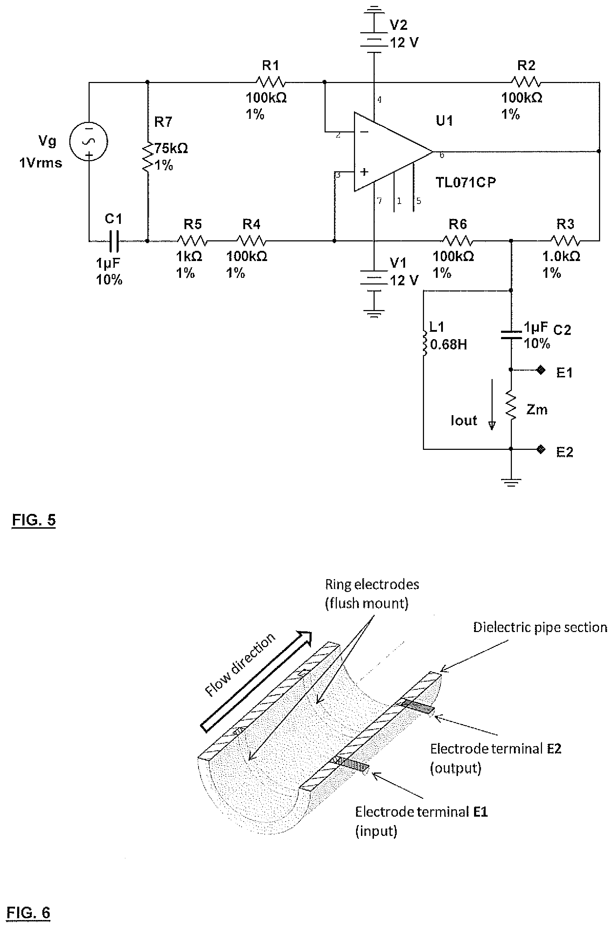

For example, 1 V of the voltage source is transformed (converted) into 1 mA current excitation passing through the electrode E1. In the case that it is considered necessary, the VCCS gain could be adjusted -increased or decreased- at any desired value. It is known from electrical circuits' theory that the current source has very high internal impedance (theoretically infinite), while the voltage source has low internal impedance (theoretically zero). Therefore the positive and the negative inputs of an operational amplifier can be used to make a current source. The VCCS is essentially a high impedance current source, and many alternative designs of current sources and VCCS exist for testing other devices and materials, or driving sensors. Most of the designs are for current sources controlled by a DC voltage source. But for the demanding application of the detection and characterization of bubbles in liquids, notably in-vitro, where the practical problems involved are not so simple or/and obvious, a versatile and reliable alternating current source (AC current) is needed, with high output impedance and wide frequency range. For these tasks, a novel, modified and improved alternating current VCCS, based on Howland Current circuit is designed, as shown in Fig. 5. The gain is set by $R_3$ and ratio of $R_1/R_2$, which is typically 1/1. In the new improved design, the ratio of $(R_5+R_4)/(R_6+R_3)$ should be matched with $R_1/R_2$ for proper biasing of the operational amplifier (Op-amp), so: The gain then is:

$$\frac{R_5+R_4}{R_6+R_3} = \frac{R_1}{R_2} \qquad (11)$$

$$|H_{VCCS}| = \frac{I_{out}}{V_g} = \frac{1}{R_3} \qquad (12)$$

Consequently, low values for $R_3$ can be used and have all the other resistors high in value, such as 100 kΩ or 1 MΩ. Based on Equation 10, the value of the resistor would be $R_3$=1kΩ, wherein the $R_3$ value may vary with the particular value of VCCS gain needed for each set of conditions, if increased detection sensitivity is required. Therefore, the output impedance of the current source is very high, and even for typical operational amplifier with CMRR of 60 dB, the output impedance would "degrade" to 10 MΩ, which is an acceptable value for applications in liquids or biomedicine. The majority of commercial operational amplifiers have CMRR (Common Mode Rejection Ratio) better than 80 dB, so the impedance is increased even more. This circuit can supply many milliamperes (or as low as microamperes) depending on the input voltage and VCCS gain. Also, with a power supply of at ±12 V DC in nominal voltage values, the maximum resulting voltages caused by the output current, can be as large as 10 V, with good efficiency. If higher resulting voltages are needed, the power supply voltage can be increased. Based on the design guidelines of Equations 11 and 12 as well as on the gain requirement of Equation 10, the resistor values selected are those shown in Fig. 5. The operation amplifier is a TL071 (but any other compatible operation amplifier can be used) JFET operational amplifier, with internal frequency compensation, high CMRR 100 dB, high slew rate 13 V/μs, low noise and distortion 0,003% (typical). Test measurements have shown that even for the case of a high value 1 kΩ load, the gain is dropped only about 0,4% and a phase shift of -5,7° is observed. But these deviations are negligible and do not affect the accuracy of the measurements because when applying the system, a baseline measurement (bubble free condition) is always taken. Therefore, these changes are known and taken into account. The measured impedance (or inversely measured admittance) as "sensed" by the electrodes E1 and E2 (Fig. 5), is the magnitude of $Z_m$ and can be simply calculated as the ratio of the differential voltage (voltage drop) between E1-E2 and output current:

$$Z_m = \frac{V_{E1} - V_{E2}}{I_{out}} = \frac{V_m}{I_{out}}$$

(13)

If the phase requirements of Equation 8 are fulfilled, then $Z_m$ is transformed to resistance, i.e. $R_m$, similarly to the case of voltage source mode.

The voltages $V_{E1}$ and $V_{E2}$ can be measured either in single ended or differential mode. In single ended mode, the voltages measured by the data acquisition card are measured with respect to the reference potential point. The most common setup is the 0 V point, or in other words the ground reference. In differential mode, the voltage difference between two potential points is measured, such as the voltage drop across a resistor. The data acquisition card can measure voltages in both differential and single ended mode and is connected to a personal computer (PC) for real time display (monitoring) and digital signal processing of the measured voltages as well. The voltage difference $V_{E1}$-$V_{E2}$ is actually the measured key parameter ($I_{out}$ is constant), because it is directly affected by the variation of two-phase's impedance, caused by the presence of bubbles. Given that in differential mode the voltage drop is measured, differential set-up seems to be a more straightforward approach for the specific application. In addition, the differential mode provides better noise and interference rejection, but it requires the front-end electronic of the data acquisition card to be carefully designed with very high and well-matched input impedances as well as opamps with high CMRR (> 80 dB). For the measurement setup, also, it is required to separate the current wires from the voltage measurement wires. Therefore, two extra cables for the voltage measurement are needed (4-wire measurement type). A typical application is shown in Fig. 6, where ring electrodes are flush mounted to the inner walls of a cylindrical pipe. The current excitation is accomplished via electrode E1, while electrode E2 is used for the voltage drop measurement.

The excitation and output voltages for the voltage source mode $V_{E1}$ and $V_{E2}$, as well as the voltage drop for the current source mode $V_{E1}$- $V_{E2}$ are recorded by the data acquisition card of high sampling rate (192 kS/s) and resolution (24 bit), providing two important advantages: a) study of electrical signals with maximum frequency in the order of several tens of kHz due to the high sampling rate and b) measurement of extremely low voltages down to microvolts due to high spatial resolution. None of the conventional electrical techniques combine the aforementioned advantages. With the present technique, the measurement sensitivity is improved several orders of magnitude, providing detection capability of pretty low volumetric gas fraction values of ~0,001%. Thus, it is possible to detect extremely small variations due to the presence of a few micro-bubbles with unprecedented sensitivity. In addition, there is no need to calibrate the output with respect to known resistors since both $V_{E1}$ and $V_{E2}$ are measured and $R_t$ (for voltage source mode) as well as $R_3$ (for the current source mode) are known. Calibration procedure is often non-linear and thus induces measurement errors which may not be negligible for the case of applications that require capturing of extremely low voltage variations.

Next, the way that the acquired signals are digitally processed employing custom-made software, that complements the hardware of the innovative electrical impedance technique, is thoroughly described. When conducting measurements of low amplitudes, it is essential to reject unwanted interference such as power line 50 Hz related noise. Therefore, the recorded AC signals $V_{E1}$ and $V_{E2}$ (or $V_{E1}$- $V_{E2}$) are digitally filtered, applying a very selective digital band pass filter centred at the excitation frequency with a nominal 3 kHz narrow bandwidth. Consequently, all kinds of electrical interference are effectively rejected, resulting in high quality electrical signals regardless of liquid physical properties, phase velocities and bubble sizes. If needed, the bandwidth can be set at any desired value, depending on the application, by changing the program code, Using a narrow filter bandwidth not only eliminates the 50 Hz related noise but also practically suppresses all extraneous noise outside the band pass frequency range. This reduces the total noise power and makes it possible to accurately measure very low voltage differentials, down to a few $\mu$V. The next critical issue of signal processing refers to the accurate extraction of acquired signals' envelope and for that reason the envelope of the filtered AC signals is digitally extracted, without any signal loss. This process is non-linear and therefore creates high frequency spurious components, which are removed by applying a low pass filter with a nominal cut-off frequency of 100 Hz. This cut-off frequency is adequate for the majority of physical characteristics of two-phase flows, but it can be set at any desired value depending on the application. It should be emphasized that conventional electrical techniques employing so far analogue electronics, either do not have filters for electrical interference rejection or these filters are not effective and selective enough, introducing in this way signal losses that affect both the accuracy and sensitivity of measurements. The particular feature of strong resistance to any form of electrical interference renders this impedance technique innovative and much more reliable than existing techniques.

The low pass filtered envelope of the signals $V_{E1}$ and $V_{E2}$, or the differential voltage $V_{E1}$-$V_{E2}$, constitutes the real peak voltage amplitude of the signals without any loss of information or distortion. Then, the impedance's magnitude of two-phase medium, $Z_m$, can be calculated employing Equations (2) and (13). Comparing $Z_m$ values prior and after gas phase entrance in the liquid phase enables the quantitative detection of bubbles inside the two-phase medium, while the resulted time series can be easily converted to volumetric gas fraction time series, for the case of a pure resistive medium -e.g. two-phase flow inside a pipe for a predefined excitation frequency-, employing standard conversion models such as Maxwell's and Begovich' ones. In any case, impedance time series carry crucial information in both frequency and time domain. Therefore, several statistical and spectral features can be associated with bubble size characteristics. As a result, various methods of signal processing and statistical analysis can be applied to the resulted time series, e.g.:

a) 1st, 2nd and higher order moments: Mean value, standard deviation, coefficient of variation, skewness and kurtosis.
b) Fast Fourier Transform (FFT) spectral analysis algorithm.
c) Auto-correlation.
d) Cross-correlation.
e) Spectrogram analysis.
f) Continuous and discrete wavelets transform.
g) Hilbert Huang transform.

Depending on the application, notably in-vitro, different quantities determined by the above statistical and spectral tools such as skewness, standard deviation, power spectral density, dominant spectral frequency, or a combination of them, can provide for the first time valuable information on bubble size distribution, as well as to be correlated with bubble size. Moreover, a number of tools contribute to further study two-phase flow dynamics, e.g. the cross-correlation of the signals between two electrode pairs provides bubble velocity. It is pointed out that the previously mentioned signal analysis techniques are not exclusive. Any digital signal processing technique or method can be applied on the impedance time series in order to study bubble size features for specific two-phase flow applications.

The most important advantage of the electrical impedance technique software concerns digital processing of the acquired signals. This processing can take place either in real time or at a later time (post-processing). Moreover, new processing features can be easily added by simply changing the program code. Computer technology advance in terms of storage capacity, memory and CPU processing power allows for the storage of numerous voluminous data files. The volume of the acquired files is determined by the recording duration which is dictated by the stochastic features of the studied two-phase flow. On the contrary, the conventional electrical techniques provide mostly predefined signal processing features which are accomplished by analogue electronics, while the storing capacity is limited by the built-in memory, which in many cases cannot be upgraded, and post-processing abilities are limited by the installed electronics components and processors.

## Claims

1. Method for detecting and characterising particles including bubbles, droplets, and/or solids, in liquids comprising the following steps:

   - applying at least two sensor means (2, 3, 8, 9) esp. electrodes so as to produce electrical impedance measurements generating an electrical signal from approximately 1 to 96 kHz,
   - acquiring electrical signals through said electrodes by means of data acquisition means (5)
   - calculating impedance time series of said electrical signals by applying digital signal processing,

   **characterised by** further steps consisting of generating said electrical signal at various frequencies by using frequency scanning through said electrodes, on the one hand, and real time monitoring by means of a personal computer (6), storing and digital processing of said time series, on the other hand, particularly for industrial in-vitro applications, wherein said particles are detected and their size and volumetric concentration are estimated.

2. An electrical impedance spectroscopy method, in particular according to claim 1, for non-invasive bubbles detection as well as estimation of their size and corresponding gas volumetric fraction, comprising the steps of:

   - attaching at least two non-invasive sensors, particularly electrodes;
   - acquiring electrical impedance measurements by means of a data acquisition card of high sampling rate and resolution;
   - real time monitoring by means of a PC, storing and digital processing of acquired signals, in particular for the detection and characterisation of bubbles in liquids,

   **characterised in that** it further comprises the steps of:

a) attaching at least two electrodes (2, 3, 8, 9) in a manner to obtain electrical impedance measurements.

b) generating an electrical signal from about 1 kHz to about 96 kHz, and

c) acquiring electrical signals through said electrodes (2, 3, 8, 9) by means of data acquisition means (5), particularly of the card type,

d) calculating impedance time series of said electrical signals by applying digital signal processing, further comprising the steps of

e) generating said electrical signal at various frequencies by using frequency scanning through said electrodes (2, 3, 8, 9) and

f) real time monitoring by means of a personal computer (6), storing and digital processing of said time series, for industrial applications in-vitro, wherein said bubbles are detected and their size and volumetric concentration are estimated.

3. Method according to claim 1 or 2, **characterised in that** the velocity of said bubbles, resp. particles is estimated and/or the presence of a few bubbles, resp. particles corresponding to extremely low volumetric concentration values is detected up to less than 0,1 %.

4. Method according to one of the claims 1 to 3, **characterised in that** a high measurement sensitivity of up to 0,001%, is performed in conjunction with a selected, resp. appropriate signal analysis, wherein a qualitative and quantitative information on bubble, resp. particle size characteristics is provided, wherein said generation of electrical signals is performed with a voltage source (1) or with a current source (6);

particularly wherein the reactance X, as the imaginary part of the impedance $Z_m$ is minimized by reducing $Z_m$ to a small amount compared to the resistance R, as the real component of $Z_m$, whereas the phase shift of the output relative to the input current is neglected, wherein the reactance is made less than 1/10 of the resistance

$$\frac{X}{R} \leq \frac{1}{10}$$

whereas the magnitude of the impedance is:

$$Z = \sqrt{R^2 + X^2} = \sqrt{R^2 + \frac{R^2}{100}} = R\sqrt{1,01} = 1,0049R \approx R$$

yielding that the impedance is transformed to resistance and

$$R_m = (k^{-1} - 1)R_t$$

yielding by equivalence, the corresponding phase shift requirement as:

$$\varphi \leq tan^{-1}\left(\frac{X}{R}\right) = tan^{-1}\left(\frac{1}{10}\right) \tag{7}$$
$$\tag{8}$$
$$-6° \leq \varphi \leq 6°,$$

wherein the phase response analysis is independent from the excitation technique and the kind of source voltage or current, more particularly whereby a frequency scanning is conducted to identify the phase response of the liquid with respect to frequency, by applying digital signal analysis and the phase shift is calculated for any discrete frequency, revealing the phase response of the liquid, which is followed by setting the excitation frequency of the signal generator in the frequency range that satisfies the phase shift requirement of said Equation (8), therefore indicating the range of maximum bubble detection sensitivity.

5. Method according to one of the preceding claims 1 to 4, **characterised in that** at least two electrodes (2, 3, 8, 9) of wire, ring, or plate type are applied.

**EP 4 018 924 A1**

6. Method according to any one of the claims 1 to 5, **characterised in that** the spectral content of said acquired signals is determined and correlated with the bubble, resp. particle size by means of an advanced signal processing technique, wherein impedance time series are analysed applying digital signal processing and statistical analysis, including any one of autocorrelation, cross-correlation, spectrogram analysis, continuous and discrete transform and Hilbert-Huang transform techniques,
and/or **in that** a cross-correlation of the signals between two electrode pairs is carried out providing bubble velocity.

7. Method according to any one of the claims 1 to 6, **characterised in that** each electrode (8, 9) of said at least two electrodes (2, 3, 8, 9) in in-vitro applications is non-intrusively flush mounted to the inner of the pipe with walls notably cylindrical or rectangular.

8. Use of the method according to one of the claims 1 to 7, for the non-intrusive estimation of the concentration of each liquid phase in a flow consisting of two immiscible liquids; and/or for the study and/or development of emulsion stability and foam drainage in food industry and cosmetics;
and/or for the crack or/and pocket detection and relevant humidity estimation in construction materials.

9. Device for the detection and characterisation of bubbles in liquids, resp. particles such as droplets, solids, in particular for carrying out a method as defined in any one of the claims 1 to 8, **characterised in that** said device comprises in mutual combination:

   - an electrical signal generator means (1),
   - at least two sensor means (2, 3, 8, 9), in particular electrodes,
   - a data acquisition device means (5),
   - a personal computer means (6) for real time monitoring, storing and digital processing of acquired signals;

   particularly wherein it further comprises a voltage source (1) and a termination resistor (4) for providing adjustment of the measurement sensitivity by means of said electrical signal generator or a current source (6) and a current sense resistor (7) by means of said electrical signal generator;
   or a voltage controlled current source (6) by means of said electrical signal generator, based on an electronic circuit (10).

10. Device according to claim 9 esp. when it depends on claim 4, **characterised in that** the data acquisition device is provided with a resolution of 24 bit and a sampling frequency of 192 kS/s by means of said device that measures gas fractions of 0,1% with a measurement sensitivity of 0,001%, esp. based on a phase shift I $\varphi$ I $\leq$ 6°.

11. Electrical impedance spectroscopy device for carrying out a method as defined in any one of the claims 1 to 7, wherein detection means (2, 3, 4, 5, 8, 9) are provided for a non-invasive detection of bubbles or particles, such as droplets, solids, as well as for the estimation of their size and corresponding volumetric concentration, **characterised in that** said detection means (2, 3, 8, 9..) consist of

   - at least two non-intrusive sensors (2, 3, 8, 9..), particularly electrodes of the flush-mounted ring, or plate type, notably depending on the pipe cross-section such as circular or rectangular, for conducting electrical measurements, and **in that** it comprises further:
   - a data acquisition means (5) consisting of a data acquisition card of high sampling rate and resolution for acquiring electrical impedance measurements;
   - monitoring means (6) consisting of a PC for real time monitoring, storing and digital processing of acquired signals.

12. System for the detection and characterization of bubbles in liquids, required in an industrial process, **characterized in that** it involves an electrical impedance spectroscopy device as defined in any one of the claims 10 to 12, for carrying out a method as defined in any one of the claims 1 to 8, wherein said method is based on the electrical impedance variation of two-phase mixtures -liquid as conductive phase, resp. gas as non-conductive phase- that is measured by non-invasively applying an AC excitation signal of a proper frequency value identified by preliminary frequency scanning, wherein said system combines signal acquisition device as hardware, and digital signal processing methods as software, thereby managing to detect accurately the presence of a few bubbles corresponding to extremely low volumetric gas fraction values, up to < 0,1%, with a high measurement sensitivity of 0,001% in conjunction with advanced signal processing, thereby providing valuable qualitative and quantitative information on bubble size characteristics and allowing for correlating bubble size to the spectral content of acquired signals.

**13.** System according to claim 12, **characterised in that** it implements an electrical technique measuring the electrical impedance, wherein the electrical impedance technique consists of a hardware in accordance with said device, and a corresponding program code as a software for digital processing of measurements in accordance with said method, wherein the electrical quantities that are measured are the resistance -or the conductance- and the reactance or both of them, as the impedance, wherein it is based on measuring the variation of electrical impedance of a two-phase mixture, consisting of a liquid being a conductive phase, and a gas as a non-conductive phase, by applying a high frequency alternating electric current (AC) or voltage via said electrodes, wherein

in a next step, the acquired signals are digitally processed employing custom made software that complements said hardware, wherein measurements of low amplitudes are conducted, wherein unwanted interference including power line related noise are rejected, wherein the recorded AC signals $V_{E1}$ and $V_{E2}$ or $V_{E1}$-$V_{E2}$ are digitally filtered, thereby applying a very selective digital band pass filter centred at the excitation frequency with a nominal narrow bandwidth of preferably about 3 kHz, thereby rejecting said electrical interferences, resulting in high quality electrical signals regardless of liquid physical properties, phase velocities and bubble sizes, wherein the bandwidth is set at a desired value depending on the application, by changing the program code, wherein a narrow filter bandwidth is used, which eliminates said power line related noise and suppresses all extraneous noise outside the band pass frequency range, thereby reducing the total noise power and allowing to accurately measure very low voltage differentials, down to a few $\mu$V, wherein the next step in said signal processing consists of the extraction of acquired signals envelope, whereby the envelope of the filtered AC signals is digitally extracted, virtually without any signal loss, wherein the process is non-linear and thus creating high frequency spurious components, which are removed by applying a low pass filter with a nominal cut-off frequency, particularly of about 100 Hz.

**14.** System according to claim 12 or 13, **characterised in that** the low pass filtered envelope of the signals $V_{E1}$ and $V_{E2}$, or the differential voltage $V_{E1}$ - $V_{E2}$, constitutes the real peak voltage amplitude of the signals without any loss of information or distortion, after which the impedance's magnitude of two-phase medium ($Z_m$) is calculated as

$$Z_m = (k^{-1} - 1)R_t \qquad (2)$$

wherein $Z_m$ is the equivalent impedance as measured by the two electrodes, resp.

$$Z_m = \frac{V_{E1} - V_{E2}}{I_{out}} = \frac{V_m}{I_{out}} \qquad (13)$$

wherein the measured impedance -or inversely measured admittance- as sensed by the electrodes (E1) and (E2), is the magnitude of $Z_m$ and is calculated as the ratio of the differential voltage -voltage drop- between (E1-E2) and output current; wherein the $Z_m$ values are compared prior and after gas phase entrance in the liquid phase, by means whereof bubbles are detected quantitatively inside the two-phase medium, while the resulted time series is converted to volumetric gas fraction time series, in particular a pure resistive medium, such as a two-phase flow inside a pipe for a predefined excitation frequency, by means of a standard conversion model of Maxwell and/or Begovich wherein impedance time series carry information in both frequency and time domain, wherein a plurality of statistical and spectral features are associated with bubble size characteristics, wherein a signal processing and statistical analysis techniques are applied to the resulting time series, such as 1st, 2nd and higher order moments, Fast Fourier Transform (FFT), Auto-correlation, Cross-correlation, Spectrogram analysis, Continuous and discrete wavelets transform, Hilbert Huang transform, wherein said signal analysis technique is applied on the impedance time series after which bubble size features are investigated targeted for two-phase flow application; wherein, depending on the application notably in-vitro, different quantities are determined by the said statistical and spectral tools, which quantities provide information on bubble size distribution, as well as to be correlated with bubble size; preferably wherein a cross-correlation of the signals between two electrode pairs is carried out providing bubble velocity.

**15.** System according to claim 13 or 14, **characterised in that** said electrical impedance technique software of digital processing of the acquired signals is carried out either in real time or at a later time as post-processing, wherein new processing features are added by changing the program code, wherein storage capacity, memory and CPU processing power are provided for the storage of numerous voluminous data files, and the volume of the acquired files is determined by the recording duration which is dictated by the stochastic features of the subject two-phase flow.

FIG. 1

FIG. 2

FIG. 3

FIG. 4

FIG. 5

Ring electrodes
(flush mount)

Dielectric pipe section

Flow direction

Electrode terminal E2
(output)

Electrode terminal E1
(input)

FIG. 6

Europäisches
Patentamt
European
Patent Office
Office européen
des brevets

## EUROPEAN SEARCH REPORT

Application Number

**EP 21 20 7579**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MOHAN M ET AL: "Investigation of a Bubble Detector based on Active Electrolocation of Weakly Electric Fish", JOURNAL OF PHYSICS: CONFERENCE SERIES, INSTITUTE OF PHYSICS PUBLISHING, BRISTOL, GB, vol. 434, no. 1, 18 April 2013 (2013-04-18), page 12088, XP020240827, ISSN: 1742-6596, DOI: 10.1088/1742-6596/434/1/012088 | 1-3,5-8 | INV. A61B5/0537 A61B8/08 A61B5/145 A61B5/1477 |
| Y | * 2. Principle of the bubble detector * | 9-15 | |
| A | * 3. Experiments * * figure 1 * | 4 | |
| | ───── | | |
| Y | US 2011/068807 A1 (KESIL BORIS [US] ET AL) 24 March 2011 (2011-03-24) * paragraph [0008] – paragraph [0015] * ───── | 9-15 | |
| | | | TECHNICAL FIELDS SEARCHED (IPC) |
| | | | A61B |

The present search report has been drawn up for all claims

| Place of search | Date of completion of the search | Examiner |
|---|---|---|
| The Hague | 19 May 2022 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 7579

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2022

| Patent document cited in search report | | Publication date | Patent family member(s) | | Publication date |
|---|---|---|---|---|---|
| US 2011068807 | A1 | 24-03-2011 | AU | 2010298385 A1 | 12-04-2012 |
| | | | CA | 2775144 A1 | 31-03-2011 |
| | | | CN | 102575998 A | 11-07-2012 |
| | | | EP | 2480878 A1 | 01-08-2012 |
| | | | IL | 218707 A | 29-02-2016 |
| | | | JP | 5666597 B2 | 12-02-2015 |
| | | | JP | 2013512414 A | 11-04-2013 |
| | | | KR | 20120074295 A | 05-07-2012 |
| | | | NZ | 598827 A | 28-03-2014 |
| | | | RU | 2012116010 A | 27-10-2013 |
| | | | US | 2011068807 A1 | 24-03-2011 |
| | | | WO | 2011038003 A1 | 31-03-2011 |

EPO FORM P0459

For more details about this annex : see Official Journal of the European Patent Office, No. 12/82

**EP 4 018 924 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6813944 B2
- US 7661293 B
- US 7661294 B